# EUROPEAN PATENT APPLICATION

(11) **EP 3 391 923 A1**
(43) Date of publication of application: **24.10.2018**
(21) Application number: 18167149.6
(22) Date of filing: 13.04.2018
(51) Int. Cl.: A61M 5/42, A61F 13/40

(54) **SELF-ANESTHETIZING HYPODERMIC NEEDLE SYSTEM AND METHOD OF USING SAME**

(30) Priority: 18.04.2017 US 201715490548
(71) Applicant: Chu, Emily, Claremont, CA 91711 (US); Chu, C. Perry, Claremont, CA 91711 (US)
(72) Inventor: Chu, Emily, Claremont, CA 91711 (US); Chu, C. Perry, Claremont, CA 91711 (US)
(74) Representative: Padial Martinez, Ana Belen

(57) **Abstract**

A self-anesthetizing needle system (10) which includes preferably an opaque tubular member (12) within which the hypodermic needle is positioned and also includes a chamber (16) at the opposite end of the tubular member within which a sponge-like material (26) is carried and which is saturated with a topical anesthetic such as ethyl chloride and includes a closure (28) to provide an airtight, water-tight seal within which the sponge-like material is contained until it is time for an injection to be given, at which point the closure member is removed and the sponge-like material is applied against the skin of a patient to whom an injection is to be administered to anesthetize the skin at the point where the injection is to be given after which the injection is then administered.

## Description

### FIELD OF THE INVENTION

This invention relates to medical equipment and more specifically to a hypodermic needle system to anesthetize the skin, hide the hypodermic needle from view of a patient and to provide safety after use.

### BACKGROUND OF THE INVENTION

A hollow pointed stainless steel needle in combination with traditional syringes is the common tool used in the healing arts to administer injections to patients. It is common knowledge in the healing art that many patients, particularly children, have fear and apprehension of receiving injections and such is emphasized when the patient is able to view the needle attached to the hypodermic syringe. Preventing such fear or apprehension will elevate the quality of work of the medical fields, thereby improving the overall health standards of the patients. Furthermore, if the patient has no fear or apprehension of an injection by a hypodermic syringe, it is much easier for the physician to perform his professional services.

As a result, there are numerous types of devices which are utilized to hide the needle from the patients. Typical of such devices are those shown in patents 3,299,891; 5,232,456; 5,634,906 and Published patent application U.S. 2012/0061286.

In addition to the foregoing, the sharply pointed end of the needle is extremely dangerous and accidental pricking is very common, even among nurses and other trained personnel. If accidental pricking occurs after the needle has been extracted from a patient with contaminated body fluid, the patient's disease may be transmitted to the treating physician or nurse. Procedures have been established for the recapping and safe disposal of medical needles immediately after their use, but the recapping process itself can be hazardous, especially when occurring in the traumatic surroundings of a first aid treatment at the site of an accident or in the context of an emergency room intervention. Various recapping systems have been devised in the past, but have not proven to be completely safe in that the needle is not automatically locked within protective sheaths or the like after use in all cases.

In addition to the foregoing, even if a needle is hidden from view of the patient, particularly a child, when the injection is given, there is initial pain associated with the injection of the needle into the patient's skin. This pain is something that is remembered by a patient, particularly a child, and even though the needle is hidden, it nonetheless instills fear and apprehension when additional injections are to be given.

There is thus a need for a hypodermic needle system which can be utilized to eliminate the pain at the time an injection is given, that will hide the needle from the view of the patient and will also provide complete containment of the needle in a protective manner after an injection is given to thereby increase the safety to the medical personnel administering the injection.

### SUMMARY OF THE INVENTION

The invention is a self-anesthetizing hypodermic needle system which includes a tubular member having first and second ends and defining a chamber at the first end carrying a sponge-like material saturated with topical anesthetic, a hypodermic needle positioned at the second end of the tubular member, and the sponge-like material being adapted to be placed in contact with the skin of a patient to whom an injection is to be administered to anesthetize the skin after which the needle is inserted and the injection is administered.

An additional feature of the present invention is that the hypodermic needle is suspended within an opaque tubular member which includes a disk-like member sealed to the inner surface of the tubular member and dividing the tubular member into first and second chambers with an air-right, water-tight sealed receptacle seated in the second chamber and housing the sponge-like material saturated with the topical anesthetic and having a removable closure member which, when removed, exposes the sponge-like material which can be applied to the skin of a patient to whom an injection is to be administered.

The method of administering an injection to the patient utilizing the hypodermic needle system of the present invention includes providing a hypodermic needle suspended within a tubular member carrying a sponge-like material saturated with a topical anesthetic contained within a sealed receptacle having a removable closure member, removing the removable closure member to expose the sponge-like material, contacting the skin of a patient with the sponge-like material for a period of approximately five seconds to allow the topical anesthetic to anesthetize the skin at the point where the injection is to be administered, inserting the needle into the skin of the patient and administering the injection, and withdrawing the needle from the skin of the patient.

### BRIEF DESCRIPTION OF THE DRAWINGS

Figure 1 is a cross-sectional view of a needle system constructed in accordance with the principles of the present invention;
Figure 2 is a cross-sectional view illustrating the administration of an injection to a patient;
Figure 3 is an exploded view showing the structure of Figure 1 in greater detail;
Figure 4 is a cross-sectional view of a needle system constructed in accordance with the principles of the present invention having an opaque cylinder hiding a needle;
Figure 5 is a cross-sectional view of the needle system of the present invention taken about the lines 5-5 of Figure 4;
Figure 6 is a plan view of a piston which is utilized in the needle system of the present invention;
Figure 7 is a perspective view illustrating construction of the tubular membeer utilized in the needle system of the present invention;
Figure 8 is a perspective view including a partial cross section illustrating the needle system of the present invention with a traditional syringe connected thereto; and
Figure 9 is a partial cross-sectional view illustrating the hypodermic needle being locked in position after use.

### DETAILED DESCRIPTION OF THE PREFERRED EMBODIMENTS

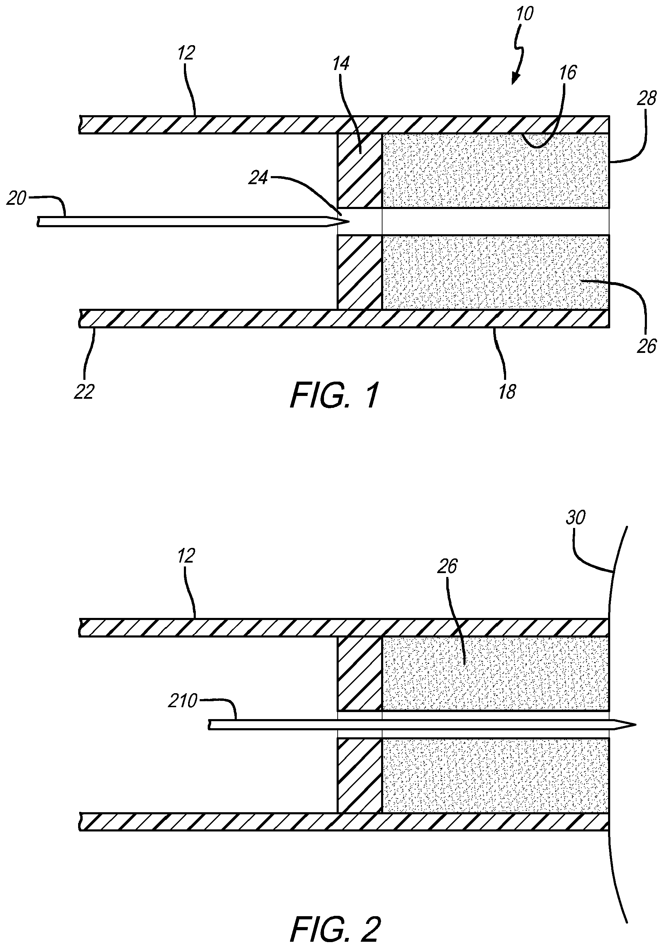

Referring now more particularly to Figures 1 and 2, there is illustrated in cross section a hypodermic needle system 10 constructed in accordance with the principles of the present invention. As is therein shown, the hypodermic needle system 10 includes a tubular member 12 which has a disk 14 secured to an internal surface of the tubular member 12 and as a result defines a chamber 16 at a first end 18 of the tubular member 12. A needle 20 is positioned at the second end 22 of the tubular member 12. The disk 14 defines a central opening 24 therethrough which is utilized to guide the needle 20 at the time that an injection is to be given. Seated within the chamber 16 is a sponge-like material 26 which is saturated with a topical anesthetic. The term "sponge-like material" as used herein is defined as any open cell reticulated foam material that becomes saturated with its surrounding elements and may be natural or synthetic. That is, the sponge-like material may be a natural occurring sponge or, alternatively, may be constructed from a synthetic material such as a polyether of various types, for example, a polyurethane sponge. So any open cell reticulated foam material which may be capable of becoming saturated with a liquid would respond to the term "sponge-like material". A closure member 28 is secured over the terminus of the first end 18 and closes the chamber 16. The closure member 28 may be any type of closure desired which will close off and seal the chamber 16 to retain the sponge-like material and the topical anesthetic held by the sponge-like material. For example, the closure member 28 may be a cap or a peel-off foil such as that utilized on some medication bottles or nutritional supplement bottles.

When an injection is to be administered to the patient, the closure member 28 is removed which causes the sponge-like material to extend from the chamber and the terminus of the end 18 of the tubular member 12 in an amount of approximately a centimeter. This then allows the surface of the sponge-like material 26 containing the topical anesthetic to be applied to the skin 30 of the patient at the point where the injection is to be administered. The saturated sponge-like material is held in place for a period of approximately 5 seconds to cause the topical anesthetic contained in the sponge-like material 26 to anesthetize the skin of the patient at the point where the injection is to be administered. After that period of time, the needle 20 is then urged forward and into and through the skin 30 of the patient and the injection administered. As a result of the contact of the skin 30 of the patient by the sponge-like material saturated with the topical anesthetic, the skin in the area where the injection is to be administered is anesthetized and thus when the needle 20 is inserted into the patient through the skin 30, the patient does not experience any pain.

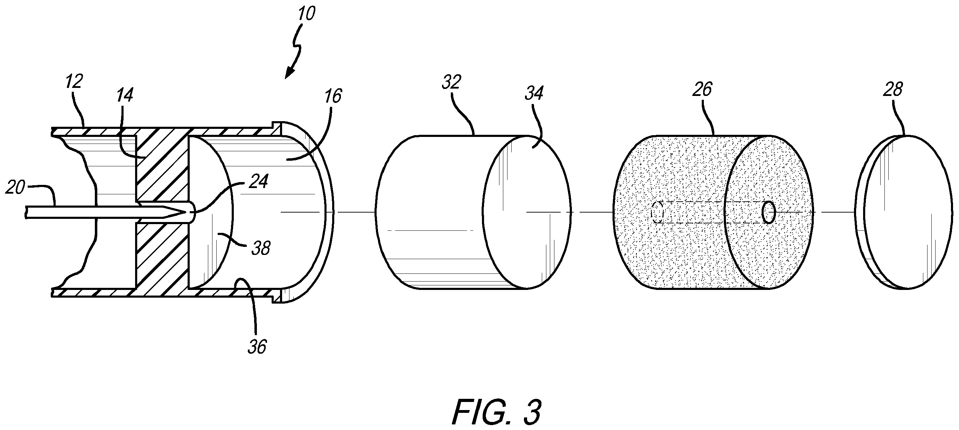

Referring now more particularly to Figure 3, there is illustrated in a perspective, partially cross-sectional view, an exploded view of the hypodermic needle system as illustrated in Figure 1. As is illustrated in Figure 3, the tubular member 12 is preferably constructed of molded plastic. Also in accordance with the principles of the present invention, the molded plastic tubular member 12 is preferably opaque and, therefore, will totally hide the needle from the patient to whom the injection is to be given. The disk 14 in accordance with a preferred embodiment of the present invention, is an integral member of the molded plastic tubular member 12 and is molded with the tubular member simultaneously when it is formed.

Also in accordance with a preferred embodiment of the present invention, the system includes a cup-like member 32 constructed of a material that is not reactive with the topical anesthetic which is saturated in the sponge-like material 26. In accordance with a preferred embodiment, the cup 32 is formed of aluminum foil or other similar foil material which will not react to the topical anesthetic, preferably ethyl chloride. Alternatively, the cup 32 could be formed from other materials and the inner surface 34 thereof may then be coated with a material which is non-reactive to the topical anesthetic carried by the sponge-like material 26.

In a further alternative embodiment, the inner surface 36 of the chamber 16 which would include the surface 38 of the disk 14 could also be coated with a material which is non-reactive to the topical anesthetic carried by a sponge-like material 26. If such is utilized, the opening 24 in the disk 14 must be sealed to provide a water-tight, air-tight chamber for receiving the sponge-like material saturated with the topical anesthetic. The material effecting the seal must be such that the needle can penetrate it without damage to the needle.

In any event, the sponge-like material is carried by the tubular member 12 within the chamber 16 and after it is so situated, the closure member 28 is secured to provide a water-tight, air-tight seal containing the sponge-like material 26 saturated with the topical anesthetic. As above indicated, the closure member 28 may be a cap or the peel-off foil such as that utilized on some medication bottles or nutritional supplement bottles.

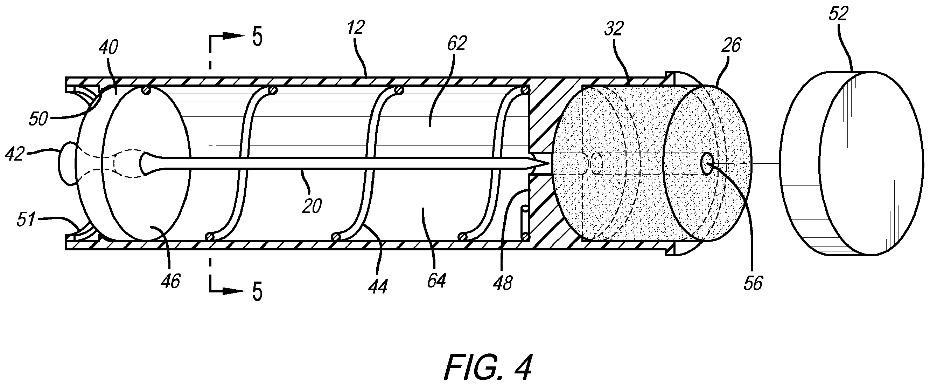

Referring now more particularly to Figure 4, the preferred embodiment of the hypodermic needle system constructed in accordance with the present invention and utilizing an opaque tubular member is illustrated. As is therein shown, the tubular member 12 includes a piston 40 which may be constructed of molded plastic that is disposed internally of the tubular member 12. The hub 42 of the needle 20 is incorporated into the piston 40 and allows a typical state-of-the-art syringe to be attached to the needle. The piston moves downwardly inside of the cylinder acting like a plunger to keep the needle moving straight into the opening 24 in the disk 14. The inner surface of the tubular member 12 includes a plurality of inwardly directed ribs two of which are shown at 62 and 64. These ribs seat within grooves formed in the piston 40 to prevent the piston from rotating and particularly to allow a doctor or nurse to attach a hypodermic syringe to the hub 42 of the needle. Without the ribs and the grooves interacting with each other an attempt to attach a syringe to the hub of the needle would merely cause the piston to rotate within the tubular member 12. In addition thereto, a spring 44 is seated against the surface 46 of the piston 40 at one end thereof and against the surface 48 of the disk 14 at the other end. This spring urges the piston upwardly against an integral lock tab system 50. An inwardly directed flange 51 functions in cooperation with the lock tab system 50 to secure the needle after use as will be described in greater detail hereinbelow. The sponge-like material 26 is contained within a cup-like member 32 as above described and the end of the tubular member 12 is closed by a closure member in the form of a cap 52 which may be secured to the end of the tubular member by threads or any other well-known system which will permit the closure member to be removed. When the closure member is removed, the sponge-like material 26 will protrude from the tubular member 12 by approximately a centimeter so that it may be applied to the skin of the patient at the time an injection is to be given.

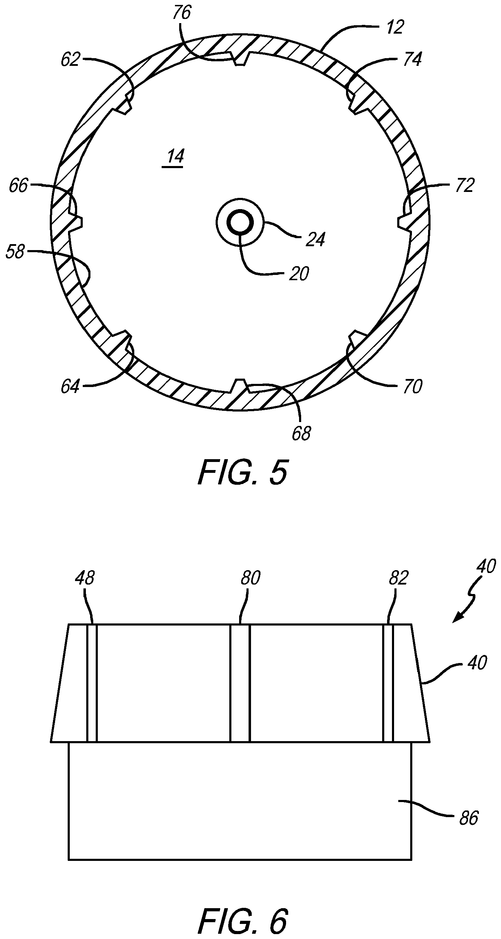

Referring now more particularly to Figure 5, there is illustrated in cross section a view of the tubular member taken about the lines 5-5 of Figure 4 but without the spring 44. As is therein shown, the internal surface 58 of the tubular member 12 has extending inwardly therefrom a plurality of ridges 62 through 76. Although eight such ridges are illustrated in Figure 5, it should be understood by those skilled in the art that that number may be varied and could include a few as two or more than eight as may be desired. The ridges seat within grooves formed in a portion of the piston and as illustrated at Figure 6 would be such as those shown at 78, 80 and 82. As illustrated in Figure 5, the needle 20 extends centrally of the tubular member 12 and extends through or toward the opening 24 in the disk 14.

Referring now more particularly to Figure 6, there is illustrated a plan view of the piston 40. As is shown, the piston 40 includes a tapered skirt 84 as well as a central body 86. As above described, the piston 40 is adapted to receive the upper portion of the hypodermic needle 20 to permit the hub 42 to extend outwardly therefrom. Also, as above described, the ridges extending from the inner surface 58 of the tubular member 12 would co-act with the grooves which are formed in the skirt 84 and as shown at 78, 80 and 82 in Figure 6 to prevent the piston from rotating within the tubular member 12 when the doctor or nurse is securing the hypodermic syringe to the hub 42 of the needle. It will be understood by those skilled in the art that although only three grooves 78, 80 and 82 are shown in Figure 6, that the additional perimeter of the skirt 84 will have additional grooves therein matching the number of ridges extending inwardly from the inner surface 58 of the tubular member 12.

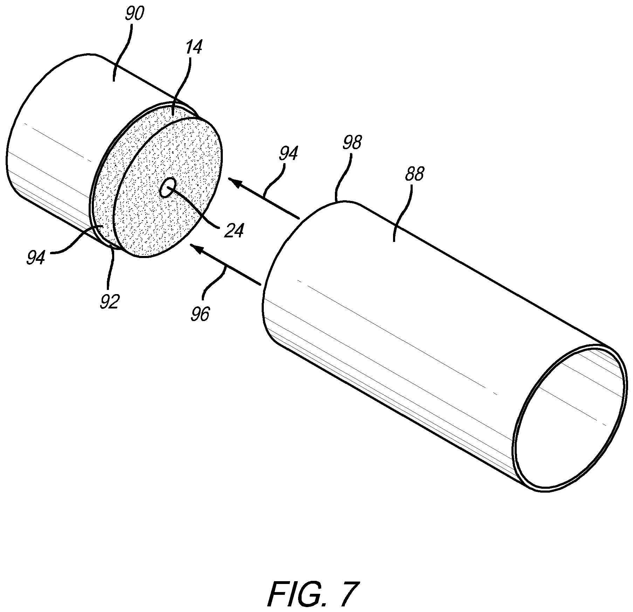

Referring now more particularly to Figure 7, there is illustrated one manner in which the hypodermic needle system of the present invention as illustrated in Figure 4 may be assembled during production. As is therein shown, the tubular member 12 may be constructed in two different sections as illustrated at 88 and 90. The section 90 would include the disk 14 having the opening 24 molded integrally with the section 90. The diameter of the disk 14 is constructed such that it is the same diameter as the internal surface of the tubular member 12 and, therefore, defines a shoulder 92 and a surface 94. When the system of the present invention, as illustrated at Figure 4, is being produced, the piston containing the needle 20 would be inserted into the section 88 of the tubular member 12 and the spring 44 would be inserted thereafter. At this point, the section 88 would be moved in the direction as shown by the arrows 94 and 96 so that the edge 98 of the Section 88 would then seat against the shoulder 92 and the inner surface of the section 88 would mate with the surface 94 of the disk 14. The two sections 88 and 90 would then be secured together either by the utilization of an adhesive which would have been applied to the surface 94 in advance of the mating or, alternatively, after the mating the surface 94 could be secured to the inner surface of the section 88 by ultrasonic welding or the application of heat and pressure as will be well known by those skilled in the art.

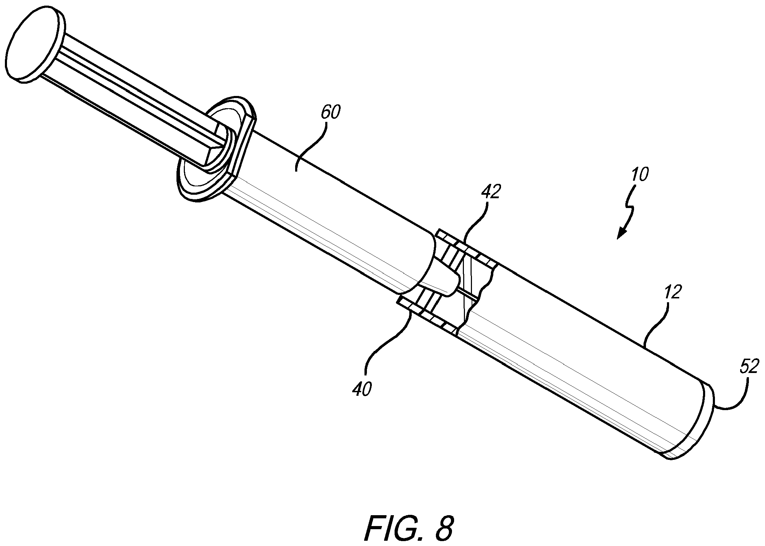

Referring now more particularly to Figure 8, which is a perspective view of the needle system 10 constructed in accordance with the principles of the present invention, and as above described to which a syringe 60 of any conventional type has been affixed to the hub 42 which is contained within the piston 40 as above described. As above described, when such occurs and the doctor or nurse is ready to administer the injection, the closure member such as the cap 52 is removed allowing the sponge-like material to protrude a centimeter or so from the end of the opaque plastic molded tubular member 12 so that the anesthetic can be applied to the skin of the patient to anesthetize the area where the injection is to be administered. The syringe 60 attached to the needle is then urged downwardly into the tubular member which as will be recognized by those skilled in the art will have a diameter sufficient to accept the barrel of the syringe 60 as it is moved downwardly to administer the injection to the patient. As above indicated, when the injection has been administered, the syringe 60 is then moved upwardly out of the tubular member 12 and disconnected from the hub of the needle and the needle system 10 is then discarded after this use, thereby containing the needle very safely internally of the tubular member 12.

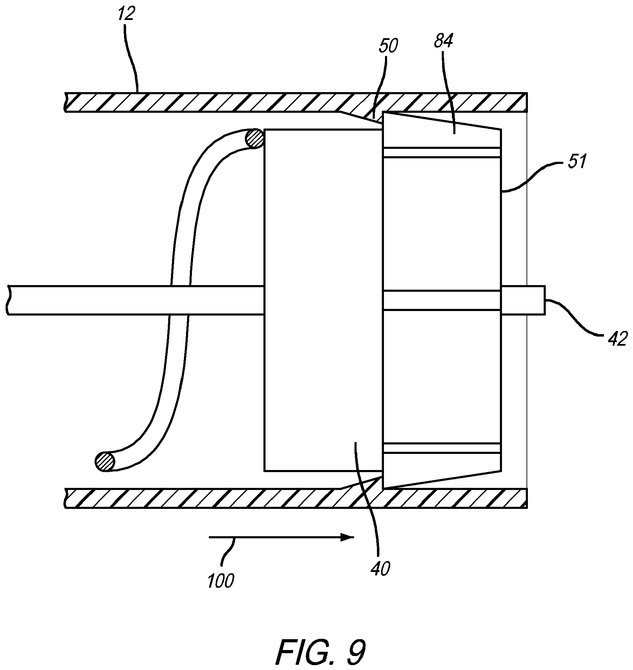

When an injection is to be given, as above indicated, the syringe 60 is affixed to the hub 42 of the needle and at that time the attendant administering the injection will push the needle downwardly after the end of the sponge-like material has been held against the skin of the patient for approximately five seconds to anesthetize the skin of the patient and the piston 40 will move against the spring 44 and the needle will enter the skin of the patient as shown in Figure 2 and the injection administered. The nurse or doctor administering the injection will then retract the needle toward the position as shown in Figure 4 and, by adding a slight force in the direction shown by the arrow 100 as shown in Figure 9 to which reference is hereby made, will cause the skirt 84 of the piston 40 to bend slightly inwardly and pass over the locking tab system 50 and seat against the inwardly directed flange 51, thereby securely locking the piston 40 between the locking tabs 50 and the inwardly directed flange 51 to thereby prevent the piston from moving in either direction, downwardly or upwardly. As a result, the needle will be securely enclosed within the tubular member 12. Since the piston is incapable of moving in either direction, the needle will not protrude from the tubular member 12 and will be securely and permanently enclosed thereby. After the piston has been thus secured, the syringe will be retracted from the hub 42 and the hypodermic needle system of the present invention can be discarded.

There has thus been disclosed a self-anesthetizing needle system which is contained within an opaque cylinder which hides the needle from the patient to whom the injection is to be given and which contains a sponge-like material carrying a topical anesthetic such as ethyl chloride which will anesthetize the skin of the patient at the point where the injection is to be given and after the injection is given, will retain the needle permanently internally of the tubular member for safe disposal.

## Claims

1. A self-anesthetizing hypodermic needle system comprising:
(A) a tubular member having first and second ends;
(B) a chamber at said first end carrying a sponge-like material saturated with a topical anesthetic;
(C) a hypodermic needle positioned at said second end; and
(D) said sponge-like material adapted to be placed in contact with the skin of a patient to whom an injection is to be administered to anesthetize the skin after which the needle is inserted and the injection administered.

2. A self-anesthetizing hypodermic needle system as defined in claim 1 which further includes an air-tight, water-tight receptacle containing said sponge-like material seated in said chamber.

3. A self-anesthetizing hypodermic needle system as defined in claim 2 wherein said receptacle comprises a removable closure member which when removed exposes said sponge-like material.

4. A self-anesthetizing hypodermic needle system as defined in claim 2 wherein said receptacle is constructed from material which is non-reactive with the topical anesthetic.

5. A self-anesthetizing hypodermic needle system as defined in claim 4 wherein the topical anesthetic is ethyl chloride;

6. A self-anesthetizing hypodermic needle system as defined in claim 5 wherein the receptacle is constructed of aluminum foil.

7. A hypodermic needle system for use with a hypodermic syringe to anesthetize the skin, provide safety after use and hide the hypodermic needle from view of a patient to whom an injection is to be given, said needle system comprising:
(A) an opaque tubular member having first and second ends and an inner surface;
(B) a disk-like member defining a central opening therethrough, to guide the needle, said disk being sealed to the inner surface of said tubular member and disposed from said second end and dividing said tubular member into first and second chambers, said first chamber having a length sufficient to receive the whole length of said hypodermic needle;
(C) an air-tight, water-tight sealed receptacle seated in said second chamber in contact with said disk;
(D) a sponge-like material saturated with a topical anesthetic received within said receptacle; and
(E) a removable closure member at said second end of said tubular member which when removed exposes said sponge-like material.

8. A hypodermic needle system as defined in claim 7 wherein said tubular member and said disk-like member are formed as an integral molded plastic member.

9. A hypodermic needle system as defined in claim 8 wherein said closure member includes an air-tight water-tight cap secured to said second end of said tubular member.

10. A hypodermic needle system as defined in claim 8 which further includes a piston seated within said first chamber, said center of said piston adapted to receive the hub of said hypodermic needle, and a spring seated between said piston and said disk urging said piston toward said first end of said tubular member.

11. A hypodermic needle system as defined in claim 7 wherein said sponge-like material defines a centrally disposed opening therethrough for pass through of said needle when an injection is administered to said patient.

12. A hypodermic needle system as defined in claim 7 wherein said receptacle is constructed from or lined with a material that is non-reactive with the topical anesthetic.

13. A hypodermic needle system as defined in clam 12 wherein the topical anesthetic is ethyl chloride.

14. A hypodermic needle system as defined in claim 13 wherein said receptacle is constructed of aluminum foil.

15. A hypodermic needle system as defined in claim 10 which further includes locking tabs on said inner surface adjacent said first end of said tubular member adapted to engage said piston after the injection is administered to prevent the hypodermic needle from being able to advance again past the second end of the tubular member keeping the entire length of the hypodermic needle covered by said tubular member.

16. A hypodermic needle system as defined in claim 10 wherein said inner surface of said tubular member includes a plurality of inwardly directed ridges and said piston defines a plurality of grooves in an outer surface thereof, said grooves receiving said ridges to prevent rotation of said piston upon a syringe being affixed to said hub of said needle.

17. A hypodermic needle system as defined in claim 15 which further includes an inwardly directed flange at said first end of said tubular member to prevent said piston from moving out of said tubular member after said locking tabs engage said piston.

18. A method of administering an injection to a patient with a hypodermic needle connected to a traditional hypodermic syringe comprising:
providing a hypodermic needle suspended within a tubular member carrying a sponge-like material saturated with a topical anesthetic contained within a sealed receptacle having a removable closure member;
removing the removable closure member to expose the sponge-like material;
contacting the skin of a patient with the sponge-like material for a period of at least five seconds to allow the topical anesthetic to anesthetize the skin at the point where the injection is to be administered;
inserting the needle into the skin of the patient and administering the injection; and
withdrawing the needle from the skin of the patient.
